# FASCICULE DE BREVET EUROPEEN

(11) **EP 4 444 453 B1**
(45) Date de publication et mention de la délivrance du brevet: **24.09.2025**
(21) Numéro de dépôt: 22840792.0
(22) Date de dépôt: 07.12.2022
(51) Int. Cl.: B01F 23/233, B01F 27/2322, B01F 27/85, B01F 33/81, B01F 35/21, G01N 33/18, C02F 3/00, C02F 3/12

(54) **DISPOSITIF POUR RÉALISATION DE TEST D'EVALUATION DES EAUX USÉES**
VORRICHTUNG ZUR DURCHFÜHRUNG EINES TESTS ZUR BEURTEILUNG VON ABWASSER
DEVICE FOR CARRYING OUT A TEST TO EVALUATE WASTEWATER

(30) Priorité: 07.12.2021 FR 2113105
(43) Date de publication de la demande: 16.10.2024
(73) Titulaire: NANTES UNIVERSITÉ, 44035 Nantes Cedex 1 (FR)
(72) Inventeur: JOUANNEAU, Sulivan, 44035 Nantes Cedex 1 (FR); LOUINEAU, Thomas, 44035 Nantes Cedex 1 (FR); THOUAND, Gérald, 44035 Nantes Cedex 1 (FR)
(74) Mandataire: Icosa
(86) Numéro de dépôt international: PCT/FR2022/052273
(87) Numéro de publication internationale: WO 2023/105163

(56) Documents cités:
- WO-A1-92/01223
- DE-A1- 2 544 204
- US-A1- 2009 208 373

## Description

Le domaine de la présente invention concerne la filière de traitement des eaux. L'invention trouve notamment des applications dans le domaine de l'épuration biologique du traitement des eaux usées, utilisant le procédé dit « à boues activées ». Il s'agit d'un mode d'épuration par cultures libres consistant à mettre en contact les eaux usées avec un mélange riche en bactéries pour dégrader la matière organique. Une aération importante est nécessaire afin de permettre l'activité de ces bactéries. L'invention concerne un dispositif permettant de réaliser un essai de toxicité pour évaluer la qualité de l'eau et une méthode pour réaliser un essai à l'aide du dispositif.

Il est connu de l'art antérieur le test OCDE 209, le but de ce test est de fournir une méthode de dépistage rapide pour évaluer les effets de substances sur les micro-organismes des boues activées du stade biologique (aérobie) des eaux usées des usines de traitement. Les résultats du test peuvent également servir d'indicateur. Ce test vise à évaluer la qualité d'une eau au moyen d'un essai d'écotoxicité basé sur l'inhibition de la consommation d'oxygène par des boues activées. Le protocole consiste à mettre en contact des boues activées (bioindicateurs) avec un échantillon durant une période définie (3 heures). Durant cette phase, l'essai est continuellement aéré de manière à avoir un taux en oxygène dissous dans l'essai proche de la saturation. Suite à cette exposition, l'aération est stoppée et la consommation en oxygène par la biomasse est suivie au moyen d'une sonde à oxygène dissous. L'investigation porte sur la toxicité de l'intrant (eaux de lavage, eaux de process, etc.) pour la station de traitement en charge de dépolluer ces effluents. Ce test est particulièrement pertinent dans le secteur industriel pour la caractérisation toxicologique des effluents issus des filières de production. En effet, à notre connaissance, il n'existe pas d'autres tests, actuellement disponibles, permettant d'évaluer l'effet d'un effluent sur la filière de retraitement des eaux usées.

Bien que simple conceptuellement, ce test requiert une logistique lourde à assumer pour les industries en termes de matériel et de ressource humaine. La mesure d'un échantillon (en accord avec le standard OCDE 209) nécessite généralement une dizaine d'heures pour l'obtention du résultat dont 65% de temps homme.

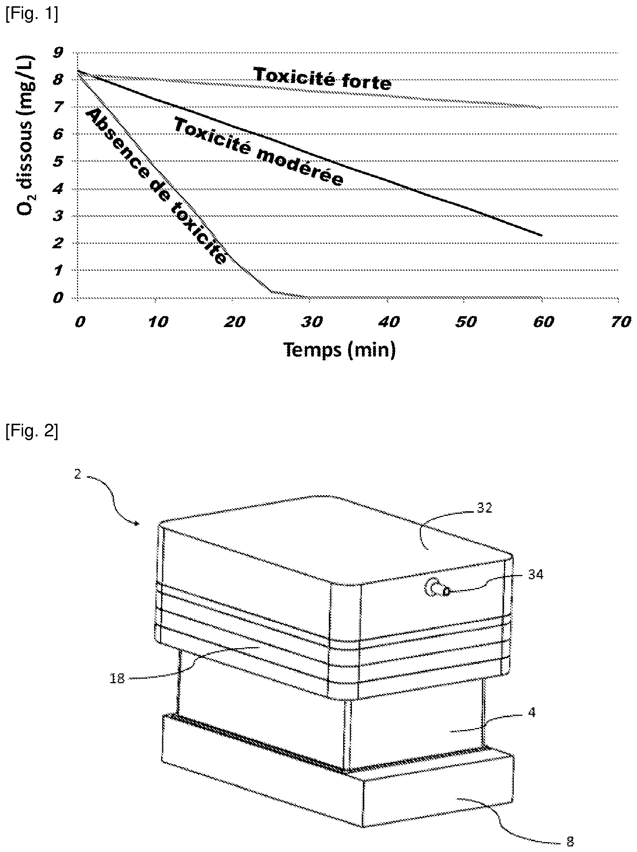

Cette méthode normée de l'art antérieur permet l'évaluation de la toxicité d'un effluent sur les microorganismes (boue activée) de station d'épuration grâce au suivi de la consommation d'oxygène (Fig. 1). Généralement ce test est réalisé en flacon de 500mL. Pour cela, 250 mL de boues activées préalablement lavées sont exposés durant 3 heures à 234 mL d'échantillon à analyser (16 mL d'eau usée standardisée sont ajouté afin de garantir un apport nutritif favorable à l'activité métabolique aérobie des microorganismes). Au cours de ces 3 heures d'exposition, le mélange est agité à l'aide d'un barreau aimanté et aéré à l'aide d'un système de bullage.

Néanmoins, cette méthode connue présente au moins deux problèmes majeurs, limitant considérablement son déploiement sur le terrain. Un premier inconvénient consiste en ce que les volumes importants usuellement utilisés pour les essais induisent un premier niveau de contrainte relatif à la mise en œuvre. En effet, la collecte et la préparation des solutions (prélèvement des échantillons, collecte et lavage des boues activées, préparation de la solution d'eau usée standardisée) requièrent du temps, de l'espace et du matériel. Il faut compter environ 45 minutes pour un flacon. Une fois préparées, les solutions sont mélangées dans un flacon de 500mL, c'est le début de la phase d'exposition de 3 heures. Une fois cette durée écoulée, l'aération est interrompue et l'activité respiratoire est suivie dans le flacon à l'aide d'une sonde à oxygène durant 40 minutes. Il est important de préciser que les volumes décrits dans la norme sont indicatifs et non obligatoires, le choix de ces volumes dépend directement du matériel disponible (notamment oxymètre).

Un second inconvénient réside sur la durée globale d'un essai. Dans la grande majorité des cas, une seule sonde à oxygène est disponible par site de traitement. Par conséquent, il est indispensable de mettre en place des créneaux décalés dans le temps (un flacon toute les 45 minutes) pour réaliser l'ensemble des tests requis par échantillon (*a minima* 3 réplicats + 1 témoin abiotique + 1 témoin inoculum + 1 témoin toxique de référence), soit une durée totale de 7,5 heures pour un échantillon (sans prendre en compte la phase de traitement des données). A cela s'ajoute le temps homme requis pour l'essai. En effet, chaque nouveau test requiert une phase préalable de préparation (lavage des boues activées, mélange des différentes solutions -soit 40 minutes par phase de préparation). Les besoins globaux en temps-homme représentent finalement environ 65% de la durée totale du test (soit environ 5 heures).

US 2009/208373 A1 divulgue les caractéristiques du préambule de la revendication 1.

A partir de cette situation, un des buts de l'invention consiste à fournir une solution nouvelle permettant de réaliser les tests de façon plus commode nécessitant moins d'espace et de réduire un temps significatif de mise en œuvre. En effet, le dispositif nécessite un espace dix fois moins important par rapport à un test classique, et ce dispositif permet de gagner du temps expérimental d'exécution du test et de mise en œuvre par l'utilisateur (temps-homme) également. Plus précisément, le temps expérimental est réduit de quatre heures par rapport au test classique connu de l'art antérieur et le temps-homme est réduit à 40 minutes versus cinq heures avec le test classique. Dès lors il est évident que cette invention présente un avantage économique non négligeable.

Pour atteindre ce but ou d'autres, l'invention propose un dispositif présentant un support réactionnel comportant une pluralité de compartiments chacun formé par une enceinte. Chaque enceinte comprenant au moins un ensemble de trois modules fonctionnels :
- un module d'agitation comprenant un arbre de rotation entrainé en rotation par un moyen d'entrainement à l'aide d'électricité et/ou à l'aide d'une arrivée de fluide, ledit arbre de rotation forme un canal apte à acheminer un fluide et selon lequel ledit arbre de rotation comprend au moins un orifice de sortie de fluide dans l'enceinte,
- un module d'aération comprenant un capillaire d'aération, et
- un module de mesure du taux d'oxygène dans l'enceinte.

Avantageusement, l'arbre de rotation présente au moins une hélice d'agitation avec au moins une pale, de préférence avec deux pales.

On peut prévoir que l'hélice d'agitation soit disposée à l'extrémité distale de l'arbre de rotation.

On prévoit que le fluide est un gaz choisi parmi le groupe constitué par l'air, l'azote, le dioxyde de carbone, l'argon, le dioxygène, et un mélange quelconque de ces derniers.

Selon un mode préféré de l'invention, le module de mesure comprend une sonde sensible à l'oxygène émettrice d'un premier signal et un capteur convertissant le premier signal en un second signal numérique analysable.

Avantageusement, cette sonde est un senseur chimique réagissant, de préférence à la concentration en oxygène dans l'enceinte. Cette sonde est émettrice d'un premier signal optique, de préférence fluorescent, et le capteur correspondant est un photo-détecteur, de préférence un fluorimètre.

Dans un mode de réalisation particulier, le dispositif tel que décrit ci-dessus est dédié pour la mise en œuvre d'une méthode d'évaluation de l'état respiratoire d'une population bactérienne dans un milieu aqueux.

L'invention concerne également une méthode de test pour évaluer la toxicité d'un échantillon aqueux mettant en œuvre un dispositif tel que décrit ci-dessus. Cette méthode comprend :
- une première étape dans chaque enceinte, consistant à ajouter plusieurs constituants : 2mL de boues « lavées », 128µL de solution nutritive, 200µL de tampon et 1,672mL d'échantillon d'eau usées ou d'eau distillée pour au moins une enceinte dite « témoin » ;
   - une deuxième étape d'agitation et d'aération à l'aide d'un moyen d'agitation et d'un moyen d'aération, pendant de préférence 3 heures,
   - une troisième étape où l'aération est arrêtée et l'agitation à une vitesse réduite est maintenue, et
   - une quatrième étape de suivi de consommation de l'oxygène durant 45min à l'aide d'un module de mesure.

Et enfin, l'invention propose également l'utilisation du dispositif et/ou de la méthode tels que décrits.

L'invention sera mieux comprise à la lecture des figures listées ci-dessous :
[Fig. 1] La figure 1 est un diagramme montrant le suivi de la cinétique de la consommation de l'oxygène d'échantillons d'effluent d'eaux usées sous les conditions du protocole expérimental recommandé par le standard OCDE 209.
[Fig. 2] La figure 2 est une vue d'ensemble du dispositif proposé selon un premier mode de réalisation de l'invention.
[Fig. 3] La figure 3 est une vue éclatée du dispositif selon le premier mode de réalisation de l'invention.
[Fig. 4] La figure 4 est une vue éclatée d'une coupe du dispositif conforme à la figure 2 selon le premier mode de réalisation.
[Fig. 5] La figure 5 est une vue en coupe en détail d'un support réactionnel conforme à la figure 2 comportant un module de mesure permettant de suivre l'activité biologique, notamment l'activité respiratoire des microorganismes.
[Fig. 6] La figure 6 est un second diagramme montrant la correspondance entre les résultats obtenues selon la méthode OCDE 209 telle que décrite dans l'art antérieur et les résultats obtenus au moyen du dispositif selon un mode de réalisation de l'invention.

L'invention a pour objet un dispositif 2 permettant la mise en œuvre de façon aisée de tests d'évaluation de milieu aqueux, s'apparentant à de l'analyse toxicologique. Ce dispositif 2 est de préférence utilisé pour la mise en œuvre d'une méthode d'évaluation de la toxicité d'un échantillon aqueux. De façon remarquable, ce dispositif 2 répond aux normes du test OCDE 209 permettant de définir des diagrammes d'analyses tel que représenté sur la figure 1. Dans le contexte de cette invention, le dispositif 2 est destiné à recevoir dans un support multi-compartimenté 4 un milieu aqueux ou un échantillon aqueux pouvant être le même ou différent dans chacun des compartiments ou enceinte 6. Ainsi dans chaque compartiment 6 une mesure de toxicité de l'échantillon ou dudit milieu aqueux est évaluée sur la base de l'inhibition de l'activité respiratoire des bactéries présent dans le milieu, il s'agit d'un indicateur de l'état physiologique des microorganismes.

Pour la compréhension générale de l'invention, la disposition de ce dispositif 2 selon l'invention est notamment visible sur la figure 2 qui est une vue générale du dispositif 2 en position fermée ou dite en position normale d'utilisation lorsque le dispositif 2 met en œuvre une méthode de test. Ce dispositif 2 selon le mode de réalisation représenté prend la forme d'un boitier compact, avantageusement facile à transporter.

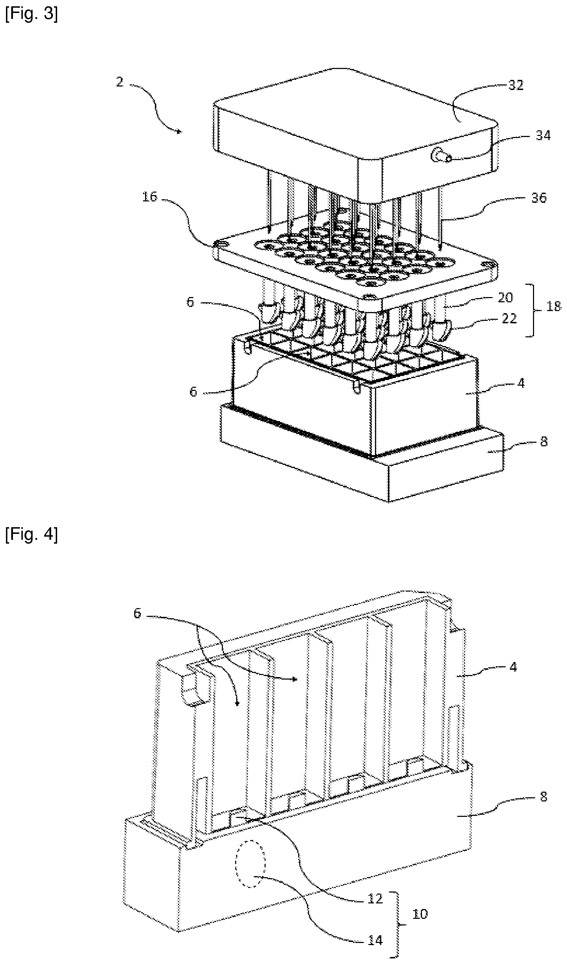

Afin de faciliter la compréhension, le dispositif 2 conforme à l'invention est représenté sur la figure 3 selon une vue générale éclatée. Sur ce mode de réalisation illustré, le dispositif 2 comprend le support réactionnel multi-compartimenté 4, formé par une pluralité d'enceintes 6. Chaque enceinte 6 est apte à accueillir environ 4mL d'échantillon. Chaque enceinte 6 à l'avantage de présenter des caractéristiques techniques permettant d'assurer un minimum d'intercontamination entre les différents compartiments d'essai. Les volumes réactionnels des échantillons aqueux ont été déterminés expérimentalement afin de minimiser les projections résultantes de l'aération et de l'agitation. Ainsi le maintien des échantillons aqueux et du fluide dans chaque enceinte 6 est directement conditionné par le respect rigoureux des paramètres suivants : volume réactionnel, dimension des enceintes 6 et couple aération/agitation. Avantageusement, selon un mode de réalisation, chaque enceinte 6 présente un col étroit à l'extrémité opposée du fond de cette dernière, pouvant être surmonté d'un capot de fermeture (non illustré) permettant également de minimiser d'éventuel risques de contaminations croisées d'échantillon aqueux d'essai entre les enceintes 6.

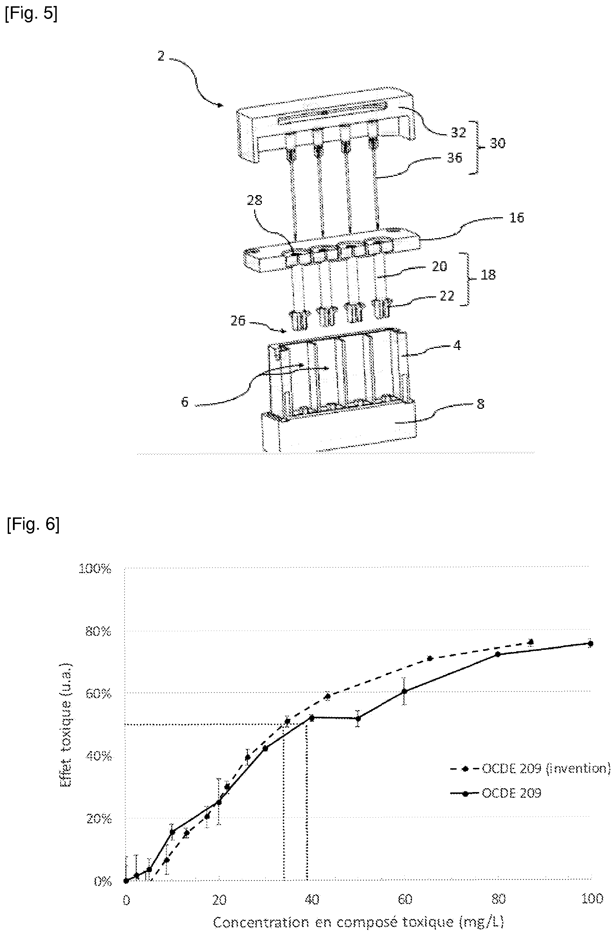

Ce support multi-compartimenté 4 est disposé sur une embase 8 comportant un ensemble de modules de mesure 10 formant ainsi le fond des enceintes 6. En condition normale d'utilisation, l'embase 8 forme la partie inférieure disposée sur un plan du type paillasse. Tels que visible sur les figures 4 et 5 l'embase 8 comprend les modules de mesure 10 selon le mode de réalisation représenté consistant en au moins un module de mesure 10 dans chaque enceinte 6. Plus précisément, chaque enceinte 6 comprend au niveau de l'embase 8 un module de mesure 10 comportant une sonde 12 destinée à être en contact avec le milieu aqueux et un capteur 14. De préférence, la sonde 12 sera une sonde à oxygène, de préférence encore, le capteur 14 sera un photo-capteur.

Comme vu précédemment la mesure de toxicité de l'échantillon ou dudit milieu aqueux est évaluée sur la base de l'inhibition de l'activité respiratoire des bactéries présent dans le milieu aqueux, il s'agit d'un indicateur de l'état physiologique des microorganismes. Le suivi de l'activité respiratoire repose sur le suivi de la concentration en oxygène dissous dans la phase aqueuse de l'échantillon ou du milieu au moyen d'une sonde à oxygène. Cette sonde 12 est destinée à être au contact du milieu à évaluer, cette dernière comprend en général deux sous-parties : un patch de mesure qui a un rôle de senseur chimique réagissant à la concentration en oxygène dans le milieu et d'émetteur d'un premier signal ce premier signal pourra être un signal optique, de préférence, fluorescent. Ce premier signal est ensuite réceptionné via le capteur 14 et selon le mode de réalisation et la composition du premier signal, de préférence il s'agit d'un photo-capteur si le premier signal est optique, de préférence il s'agira d'un fluorimètre. Ce capteur 14 réceptionne puis converti le premier signal reçu en second signal numérique analysable.

Tel que visible sur les figures 3 et 5, une plaque 16 comporte un ensemble de modules d'agitation 18. Elle est disposée sur le support réactionnel multi-compartimenté 4 à l'opposé de l'embase 8 décrite précédemment. Chaque module d'agitation 18 comprend au moins un arbre de rotation 20 et une hélice d'agitation 22, solidaire de ce dernier et disposée à son extrémité distale, c'est-à-dire à l'extrémité opposée d'un moyen d'entrainement de l'arbre de rotation 20. Ce module d'agitation 18 présente une conception destinée à prévenir le risque de contamination entre chaque enceinte 6. Plus précisément, la vitesse de rotation et le profil de l'hélice d'agitation 22 ont a été déterminés afin de limiter les projections de fluide d'essai entre les enceintes 6, et de ce fait les contaminations. L'hélice d'agitation 22 est disposée à proximité du fond de l'enceinte 6 pour optimiser un mélange homogène au plus près de la sonde 12 et ainsi pallier la problématique de sédimentation pouvant être rencontrées durant l'utilisation du dispositif 2. En effet, il a été noté que dans chaque enceinte 6 remplie d'un échantillon aqueux, une hétérogénéité importante peut apparaitre en raison d'un défaut d'agitation portant, d'une part, sur des disparités des microorganismes quant à l'accès aux nutriments et à l'oxygène et d'autre part, sur de la sédimentation des particules en suspensions dont des microorganismes. De plus, il est important de palier au risque d'apparition de zone anaérobie dans l'enceinte 6, néfaste dans le cadre du test d'évaluation de la toxicité. Dans ces deux cas, ces hétérogénéités peuvent nuire à la qualité des résultats du test.

Cette plaque 16 permet d'actionner l'ensemble des modules d'agitation 18 de chaque enceinte 6 simultanément ou individuellement sans contamination entre ces dernières. La vitesse d'agitation de chaque module d'agitation 18 est modulable afin de répondre aux contraintes de méthode d'évaluation choisie par l'opérateur.

On notera que chaque arbre de rotation 20 présente un canal 24 en son centre apte à acheminer un fluide, cet arbre de rotation 20 comprend au moins un orifice de sortie de fluide 26 dans l'enceinte 6. Il comprend également un orifice d'entrée 28 tel que visible sur la figure 5.

Le fluide peut-être un gaz choisi parmi le groupe constitué par l'air, l'azote, le dioxyde de carbone, l'argon, le dioxygène, et un mélange quelconque de ceux-ci. De préférence, le gaz sera de l'oxygène permettant ainsi d'oxygéner le milieu aqueux dans l'enceinte.

Selon le premier mode de réalisation de l'invention tel que décrit et représenté sur les figures 3 et 5, le dispositif 2 présente donc au moins un module de mesure 10, un module d'agitation 18 et un module d'aération 30, ledit module d'aération 30 est formé par une chambre de compression 32 présentant une bouche d'entrée d'air 34 reliée à une pompe d'aération et au moins un capillaire d'aération 36. Chaque capillaire d'aération 36 est destiné à aérer une enceinte 6. Selon le premier mode de réalisation présenté, l'aération est une fonction importante permettant d'assurer une oxygénation suffisante du milieu réactionnel, dans chaque enceinte 6, en accord avec les critères de la norme du test OCDE 209.

De préférence, ce module d'aération 30 tel représenté comporte une chambre de compression 32 mutualisée reliée à la pluralité de capillaires d'aération 36 répartis individuellement dans une pluralité d'enceintes 6.

La pompe d'aération assure une pression constante comprise entre 0.3-0.8 bars permettant un contrôle fiable et précis de l'apport en oxygène dans le milieu aqueux ou appelé réactionnel. Cette pression a été fixée afin d'assurer un apport suffisant en oxygène tout en évitant les contaminations entre les enceintes 6 induits par l'apparition d'émulsions. La chambre de compression 32 est une chambre étanche permettant de répartir de façon homogène le flux avant d'être envoyé dans les capillaires d'aération 36. Selon ce premier mode de réalisation, les capillaires d'aération 36 assurent une distribution homogène du fluide, de préférence de l'oxygène, dans chaque enceinte 6.

Avantageusement, le dispositif 2 comprend pour chaque enceinte 6 un système alliant donc au moins trois modules fonctionnels dédiés à la mesure du taux d'oxygène, par exemple, au contrôle de l'aération et à la mise en place d'une agitation. Ces trois modules fonctionnels sont aptes à fonctionner séparément les uns des autres. Selon un mode de réalisation, le système d'une enceinte 6 est indépendant des autres systèmes des autres enceintes 6, notamment de par leurs actionnements. Dans un autre mode de réalisation, les systèmes sont aptes à fonctionner simultanément dans l'ensemble des enceintes 6.

Pour la mise en œuvre du test OCDE 209 à l'aide du dispositif selon l'invention. Selon ce mode de réalisation, une étape préliminaire à la réalisation de la méthode mettant en œuvre le dispositif selon l'invention, consiste en un enchaînement d'étapes telles que citées ci-dessous :
- Préparation d'une solution nutritive et d'une solution tampon à pH 7,
- Lavage des « boues activées » comportant au moins un cycle de centrifugation avec extraction d'un surfactant et ajoutant d'une solution aqueuse, et
- Ajustement de la biomasse comprise dans les boues activées à la bonne concentration choisie, par exemple à 3g/L en matière sèche.

La méthode de test pour évaluer la toxicité d'un échantillon aqueux selon l'invention comprend les étapes suivantes:
- une première étape dans chaque enceinte 6, consistant à ajouter plusieurs constituants : 2mL de boues « lavées », 128µL de solution nutritive, 200µL de tampon et 1,672mL d'échantillon d'eau usées ou d'eau distillée pour au moins une enceinte 6 dite « témoin » ;
- une deuxième étape d'agitation et d'aération à l'aide du moyen d'agitation 18 et du moyen d'aération 30, pendant de préférence 3 heures,
- une troisième étape où l'aération est arrêtée et l'agitation à une vitesse réduite est maintenue, et
- une quatrième étape de suivi de consommation de l'oxygène durant 45min à l'aide du module de mesure 10, comprenant de préférence.

Le milieu réactionnel est tamponné (tampon TRIS à pH 7) afin d'éviter les modifications de pH induits par la solubilisation du dioxyde de carbone injecté à l'aide du capillaire d'aération 36 (la formation d'acide carbonique) dans le milieu réactionnel et susceptible d'induire des erreurs quant aux résultats fournis par le test.

L'ensemble du dispositif 2 est pilotable et ajustable permettant de modifier à la demande en direct ou de façon programmée certaines variables du test en fonction des besoins, telles que la vitesse d'agitation, le flux d'aération, etc.

La mise en œuvre du dispositif 2 et donc l'application de la méthode de test conformément à l'invention, nécessite une étape préliminaire de préparation de l'échantillon aqueux. En effet, des échantillons de boues activées subissent un traitement. La boue activée consiste en la mise en contact de l'eau à épurer avec une biomasse de micro-organisme. L'étape préliminaire consiste en la préparation en amont des boues activées qui doivent être lavées par centrifugation, le surnageant récolté est extrait et remplacé par une solution aqueuse de salinité adaptée à l'inoculum ou à la biomasse comprise dans l'échantillon, enfin les boues doivent être ajustée à la concentration souhaitée, par exemple pour le test spécifique OCDE 209 la concentration doit être de 3g/L._Pour ajuster la concentration, une solution tampon concentrée à un pH de 7 est préparée (Tris HCl et Trizma Base, X200). Une solution nutritive est également préparée pour répondre aux exigences de la norme OCDE 209. Les éléments sont ajoutés par tranche de 2mL de boues activée lavées, 128µL de solution nutritive, 200µL de tampon pH7 et 1,672mL de solution à analyser. Une fois, les échantillons aqueux prêts, le test peut être lancé pour 3 heures d'agitation et d'aération à environ 800mBar. A la fin de cette période de 3 heures d'exposition, l'aération est stoppée afin de permettre le suivi de la cinétique respiratoire des microorganismes au moyen des patchs positionnés au fond de chaque enceinte 6. Cette phase de mesure s'étend sur une période d'environ 45min. Tel que visible sur la figure 6, les résultats obtenus avec le dispositif 2, suite à sa mise en œuvre, ont été confrontés avec ceux obtenus par la méthode conventionnelle de l'art antérieur (tel que représenté dans la figure 1). Dans le cas par exemple du PCP, molécule utilisée pour ce test, il apparait que les valeurs obtenues à l'aide du dispositif 2 selon l'invention sont similaires aux résultats obtenus avec les conditions du test OCDE 209 de l'art antérieur. Néanmoins, l'invention propose avantageusement un gain de temps et d'espace incomparable par rapport aux conditions du test OCDE 209 de l'art antérieur. De plus à titre d'exemple, les concentrations effectrices médianes (EC50) entre les deux méthodes sont très similaires et acceptables industriellement avec une variabilité d'environ 6%, c'est-à-dire en dessous de la variabilité attendue pour le test de l'art antérieur en termes de reproductibilité qui présente au minimum une variabilité de 20%.

De plus, on notera qu'avantageusement la méthode innovante de la présente invention offre une meilleure répétabilité des résultats au vu des écarts-types bien moins importants que pour la méthode conventionnelle.

Ce dispositif selon l'invention représente dès lors le seul moyen connu à ce jour pour évaluer la toxicité d'un effluent selon le standard OCDE 209 sur les microorganismes de station d'épuration avec une vélocité et une mise œuvre remarquable par rapport à l'art antérieur.

Les modes de réalisation qui ont été décrits en détails ci-dessus ne sont pas limitatifs de l'invention. En tout état de cause, l'invention ne saurait se limiter aux modes de réalisation spécifiquement décrits dans ce document.

## Revendications

1. Dispositif (2) présentant un support réactionnel (4) comportant une pluralité de compartiments chacun formé par une enceinte (6) comprenant au moins un ensemble de trois modules fonctionnels :
- un module d'agitation (18) comprenant un arbre de rotation (20) entrainé en rotation par un moyen d'entrainement à l'aide d'électricité et/ou à l'aide d'une arrivée de fluide,
- un module d'aération (30) comprenant un capillaire d'aération (36), et
- un module de mesure (10) du taux d'oxygène dans l'enceinte (6), **caractérisé en ce que** ledit arbre de rotation (20) forme un canal (24) apte à acheminer un fluide et ledit arbre de rotation (20) comprend au moins un orifice de sortie de fluide (26) dans l'enceinte (6).

2. Dispositif (2) selon la revendication 1, selon lequel ledit arbre de rotation (20) présente au moins une hélice d'agitation (22) avec au moins une pale, de préférence deux pales.

3. Dispositif (2) selon la revendication précédente, selon lequel l'hélice d'agitation (22) est disposée à l'extrémité distale de l'arbre de rotation (20).

4. Dispositif (2) selon l'une quelconque des revendications précédentes, selon lequel le fluide est un gaz choisi parmi le groupe constitué par l'air, l'azote, le dioxyde de carbone, l'argon, le dioxygène, et un mélange quelconque de ceux-ci.

5. Dispositif (2) selon l'une quelconque des revendications précédentes, selon lequel le module de mesure (10) comprend une sonde (12) sensible à l'oxygène émettrice d'un premier signal et un capteur (14) convertissant le premier signal en un second signal numérique analysable.

6. Dispositif (2) selon la revendication précédente, selon lequel la sonde (12) est un senseur chimique réagissant, de préférence à la concentration en oxygène dans l'enceinte (6), émetteur d'un premier signal optique, de préférence fluorescent et le capteur (14) est un photo-détecteur, de préférence un fluorimètre.

7. Dispositif (2) pour la mise en œuvre d'une méthode d'évaluation de l'état respiratoire d'une population bactérienne dans un milieu aqueux selon l'une quelconque des revendications 1 à 6.

8. Méthode de test pour évaluer la toxicité d'un échantillon aqueux mettant en œuvre un dispositif (2) selon l'une quelconque des revendications précédentes, la méthode comprenant :
- une première étape dans chaque enceinte (6), consistant à ajouter plusieurs constituants : 2mL de boues « lavées », 128µL de solution nutritive, 200µL de tampon et 1,672mL d'échantillon d'eau usées ou d'eau distillée pour au moins une enceinte (6) dite « témoin » ;
- une deuxième étape d'agitation et d'aération à l'aide d'un moyen d'agitation (18) et d'un moyen d'aération (30), pendant de préférence 3 heures,
- une troisième étape où l'aération est arrêtée et l'agitation à une vitesse réduite est maintenue, et
- une quatrième étape de suivi de consommation de l'oxygène durant 45min à l'aide d'un module de mesure (10).

9. Utilisation du dispositif selon l'une quelconque des revendications 1 à 7.

## Patentansprüche

1. Vorrichtung (2) mit einem Reaktionsträger (4), der eine Vielzahl von Fächern umfasst, die jeweils aus einem Gehäuse (6) bestehen, das mindestens eine Gruppe von drei Funktionsmodulen umfasst:
- ein Rührmodul (18), das eine Drehwelle (20) umfasst, die von einem Antriebsmittel mithilfe von Strom und/oder mithilfe einer Fluidzufuhr in Drehung angetrieben wird,
- ein Belüftungsmodul (30), das eine Belüftungskapillare (36) umfasst, und
- ein Messmodul (10) für den Sauerstoffgehalt in dem Gehäuse (6), **dadurch gekennzeichnet, dass** die Drehwelle (20) einen Kanal (24) bildet, der geeignet ist, ein Fluid zu leiten, und die Drehwelle (20) mindestens eine Fluidaustrittsöffnung (26) in dem Gehäuse (6) umfasst.

2. Vorrichtung (2) nach Anspruch 1, wobei die Drehwelle (20) mindestens einen Rührflügel (22) mit mindestens einem Flügel, vorzugsweise zwei Flügeln, aufweist.

3. Vorrichtung (2) nach dem vorhergehenden Anspruch, wobei der Rührflügel (22) am distalen Ende der Drehwelle (20) angeordnet ist.

4. Vorrichtung (2) nach einem der vorhergehenden Ansprüche, wobei das Fluid ein Gas ist, das aus der Gruppe ausgewählt ist, die aus Luft, Stickstoff, Kohlendioxid, Argon, Dioxygen und einer beliebigen Mischung davon besteht.

5. Vorrichtung (2) nach einem der vorhergehenden Ansprüche, wobei das Messmodul (10) eine sauerstoffempfindliche Sonde (12) umfasst, die ein erstes Signal ausgibt, und einen Sensor (14), der das erste Signal in ein zweites auswertbares digitales Signal umwandelt.

6. Vorrichtung (2) nach dem vorhergehenden Anspruch, wobei die Sonde (12) ein chemischer Sensor ist, der vorzugsweise auf die Sauerstoffkonzentration in dem Gehäuse (6) reagiert und ein erstes optisches Signal, vorzugsweise ein Fluoreszenzsignal, aussendet, und der Sensor (14) ein Fotodetektor, vorzugsweise ein Fluorimeter, ist.

7. Vorrichtung (2) zum Durchführen eines Verfahrens zur Beurteilung des Atemzustands einer Bakterienpopulation in einem wässrigen Medium nach einem der Ansprüche 1 bis 6.

8. Testverfahren zum Beurteilen der Toxizität einer wässrigen Probe unter Verwendung einer Vorrichtung (2) nach einem der vorhergehenden Ansprüche, wobei das Verfahren Folgendes umfasst:
- einen ersten Schritt in jedem Gehäuse (6), der in dem Hinzufügen mehrerer Bestandteile besteht: 2 ml "gewaschener" Schlamm, 128 µl Nährlösung, 200 µl Puffer und 1,672 ml Probe von Abwasser oder destilliertes Wasser für mindestens einen "Kontrollraum" (6);
- einen zweiten Schritt des Rührens und Belüftens unter Verwendung eines Rührmittels (18) und eines Belüftungsmittels (30), vorzugsweise für 3 Stunden,
- einen dritten Schritt, in dem das Belüften gestoppt und das Rühren mit reduzierter Geschwindigkeit aufrechterhalten wird, und
- einen vierten Schritt des Überwachens des Sauerstoffverbrauchs über 45 min mithilfe eines Messmoduls (10).

9. Verwendung der Vorrichtung nach einem der Ansprüche 1 bis 7.

## Claims

1. Device (2) having a reaction support (4) comprising a plurality of compartments each formed by an enclosure (6) comprising at least one set of three functional modules:
- an agitation module (18) comprising a rotating shaft (20) driven in rotation by a driving means using electricity and/or using a fluid inlet,
- an aeration module (30) comprising an aeration capillary (36), and
- a measurement module (10) for measuring the oxygen level in the enclosure (6) **characterized in that** said rotating shaft (20) forms a channel (24) suitable for conveying a fluid and said rotation shaft (20) comprises at least one fluid outlet orifice (26) in the enclosure (6).

2. Device (2) according to claim 1, wherein said rotating shaft (20) has at least one agitation propeller (22) with at least one blade, preferably two blades.

3. Device (2) according to the preceding claim, wherein the agitation propeller (22) is arranged at the distal end of the rotating shaft (20).

4. Device (2) according to any one of the preceding claims, wherein the fluid is a gas selected from the group consisting of air, nitrogen, carbon dioxide, argon, dioxygen, and any mixture thereof.

5. Device (2) according to any one of the preceding claims, wherein the measurement module (10) comprises a probe (12) sensitive to oxygen emitting a first signal and a sensor (14) converting the first signal into a second analyzable digital signal.

6. Device (2) according to the preceding claim, wherein the probe (12) is a chemical sensor reacting, preferably to the oxygen concentration in the enclosure (6), emitting a first optical signal, preferably fluorescent, and the sensor (14) is a photodetector, preferably a fluorimeter.

7. Device (2) for implementing a method of evaluating the respiratory state of a bacterial population in an aqueous medium according to any one of claims 1 to 6.

8. Test method for evaluating the toxicity of an aqueous sample using a device (2) according to any one of the preceding claims, the method comprising:
- a first step in each enclosure (6), consisting of adding several components: 2 mL of "washed" sludge, 128 µL of nutrient solution, 200 µL of buffer and 1.672 mL of wastewater sample or distilled water for at least one enclosure (6) called "control";
- a second step of agitation and aeration using an agitation means (18) and an aeration means (30), preferably for 3 hours,
- a third step where aeration is stopped and agitation at a reduced speed is maintained, and
- a fourth step of monitoring oxygen consumption for 45 minutes using a measurement module (10).

9. Use of the device according to any one of claims 1 to 7.
